# EUROPEAN PATENT SPECIFICATION

(11) **EP 0 737 058 B1**
(45) Date of publication and mention of the grant of the patent: **24.02.1999**
(21) Application number: 95902647.7
(22) Date of filing: 18.11.1994
(51) Int. Cl.: A61J 1/00, A61J 1/05, A61M 39/04

(54) **FLUID PORT RESEAL MEMBER**
WIEDERVERSCHLIESSBARER VERSCHLUSS FÜR FLÜSSIGKEITSÖFFNUNG
ELEMENT POUR REFERMER D'UNE MANIERE ETANCHE UN ORIFICE A FLUIDE

(30) Priority: 28.12.1993 US 174530
(43) Date of publication of application: 16.10.1996
(73) Proprietor: Abbott Laboratories, Abbott Park, Illinois 60064-3500 (US)
(72) Inventor: FREDERICK, Waren, P., Wonder Lake, IL 60097 (US); HELGREN, R., Hayes, Mundelein, IL 60060 (US); KRUGER, Robert, J., McHenry, IL 60050 (US); LARKIN, Mark, E., Lindenhurst, IL 60046 (US)
(74) Representative: Modiano, Guido, Dr.-Ing.
(86) International application number: US9413438
(87) International publication number: WO9517873

(56) References cited:
- EP-A- 0 544 653
- WO-A-94/03373
- US-A- 5 178 607

## Description

### TECHNICAL FIELD OF THE INVENTION

This invention relates generally to a penetrable rubber reseal member used for sealing a fluid access port of a solution container or a Y-site of an infusion tubing set, and more particularly to a reseal member that can be penetrated by a blunt cannula.

### BACKGROUND OF THE INVENTION

The use of reseal members for sealing fluid access ports, particularly in containers for intravenous solutions, is well-known in the art. Reseal members have also commonly been used to seal access ports in Y-sites of infusion tubing sets and in containers of medicaments. The reseal member prevents leakage of liquid from within the container after the member is pierced by a cannula or needle to create a passage for the cannula therethrough so that fluids may be removed from the container, or be added to and mixed with the fluids in the container.

A typical prior art reseal member is comprised of a generally cylindrical, solid, rubber body. To add fluids, the reseal member is pierced by a sharp cannula or needle. Sharp cannulas or needles are commonly used to penetrate the reseal member because the reseal member is thick and solid at the insertion point.

One disadvantage of using this type of prior art reseal member is possibility of needle sticks since a sharp cannula or needle is needed to pierce the solid, rubber body. To overcome this disadvantage, sharp cannulas or needles are being replaced with blunt cannulas. However, a blunt cannula cannot be inserted in this type of reseal member without application of undesirably high force.

EP-A-544 653 describes with reference to the embodiment of its Fig. 16 an injection site usable with a blunt cannula including inter alia septum 52e with a slit 66e extending partially through the septum. The slit is formed from the front face of the septum and ends at a certain distance from the rear face of the septum. However, the injection site of EP-A-544 653 still need the application of undesirably high force to insert the cannula.

US-A-5 178 607 describes a blood aspiration assembly septum and a blunt needle aspirator. The assembly includes a receiver with a housing which is closed by a resilient plug having a recess 237 and a slit 246, wherein the slit 246 completely extends a through the axis of the plug to an upper face thereof. The slit is provided by penetrating the plug with a steel needle having an outside diameter of 1,5 mm. The device of US-A-5 178 607 does however not provide for a satisfactory solution as far as sealing is concerned.

The present invention is intended to overcome the above disadvantages as well as to present several significant advantages.

### SUMMARY OF THE INVENTION

This invention pertains to a rubber (*e*.*g*., gum rubber, silicone rubber, and the like) reseal member used for sealing a typical fluid access port, particularly in a solution container, as recited in claims 1-7. Alternatively, the reseal member may be used in a typical injection site, such as a Y-site of an infusion tubing set.

The fluid access port includes a cylindrical, peripheral wall with open ends. The reseal member is positioned within the fluid access port and is fitted in fluid tight relationship with the wall. The reseal member has an end portion positioned generally at one of the open ends of the peripheral wall so that the reseal member can be penetrated by a blunt cannula so fluids may be passed into or removed from the solution container.

The reseal member is made of rubber and includes a region which exhibits a relatively reduced resistance to penetration by the blunt cannula, relative to the remaining area of the rubber body. In the present invention, this region includes the means as defined in claim 1. To help a user place the blunt cannula in the correct position for penetration, the reseal member may include a target at the exposed end of the reseal member.

The body of the reseal member may take one of several forms. In each embodiment, the reseal member takes the form of a generally cylindrical body. According to one aspect of the invention, the reseal member includes an integral annular shoulder extending from an end of the reseal member with an integrally attached circular wall that extends around a portion of the exterior of the fluid port.

Yet according to another aspect of the invention, an end of the reseal member is dished (*i*.*e*., generally concave) to facilitate swabbing the end with an anti-microbacterial agent before the insertion of the blunt cannula. The body also includes an annular ridge for creating a radial fluid tight seal with the interior of the fluid access port.

This invention contemplates that a user may insert a blunt cannula through∼the novel reseal member of the present invention with minimal insertion force. This invention also contemplates that upon passage of a blunt cannula through the reseal member, the reseal member forms a fluid-tight seal around the cannula so as to prevent leakage of fluids therethrough. It is further contemplated that upon withdrawal of the blunt cannula, the reseal member reforms a generally fluid-tight seal (by virtue of its resilience) so fluids will not pass therethrough.

These and other objects, features, and advantages of this invention are evident from the following description of a preferred embodiment of this invention with reference to the accompanying drawings.

### BRIEF DESCRIPTION OF THE DRAWINGS

Figure 1 is an elevational view of a solution container with a side port and a down port, each including a reseal member according to the present invention, and a tubing set which can be attached to the container by a blunt cannula and which has a Y-site including a reseal member according to the present invention;
Figure 2 is a perspective view of a solution container and a ferrule cap that includes a reseal member according to the present invention;
Figure 3 is a cross-sectional view of a reseal member and an access port according to a first embodiment of the invention;
Figure 4 is a view similar to Figure 3 illustrating an embodiment different from the invention;
Figure 5 is a view similar to Figure 3 illustrating a further embodiment different from the invention;
Figure 6 is a view similar to Figure 3 illustrating a further embodiment different from the invention;
Figure 7 is a view similar to Figure 3 illustrating a further embodiment different from the invention;
Figures 8a-8b are cross-sectional views of a reseal member according to a further embodiment different from the invention;
Figures 9a-9b are views similar to Figures 8a-8b illustrating a further embodiment different from the invention; and
Figure 10 is a simplified, plan view of an exposed end of a reseal member different from the invention illustrating an area that if penetrated by a blunt cannula will allow the blunt cannula to be inserted into the reseal member.

### DETAILED DESCRIPTION OF THE PREFERRED EMBODIMENT

While the present invention is susceptible of embodiments in various forms, there is shown in the drawings and will hereinafter be described presently preferred embodiments, with the understanding that the present disclosure is to be considered as an exemplification of the invention, and is not intended to limit the invention to the specific embodiments illustrated.

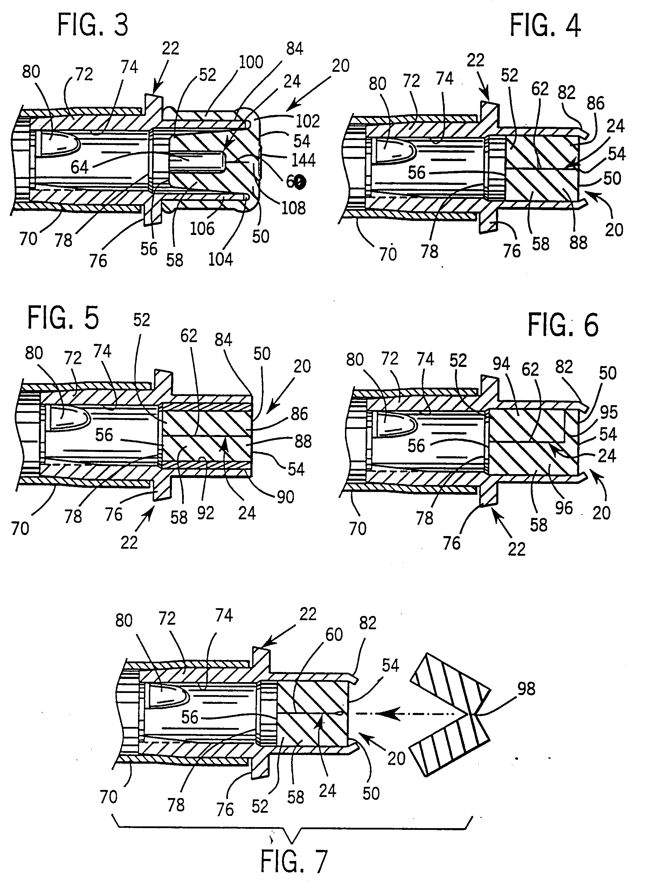

The embodiments of Figs. 4-10 (and accompanying description) are not part of the claimed invention, except for the portions of Fig. 7 (and accompanying description) which describe an elastomeric body comprised of two integrally joined halves positioned adjacent each other and joined to each other by a relatively thin portion of the elastomeric body through which a blunt cannula is insertable, as recited in claim 6. Therefore, the above mentioned embodiments (except for the teachings relative to claim 6) are envisaged as examples useful for understanding the present invention.

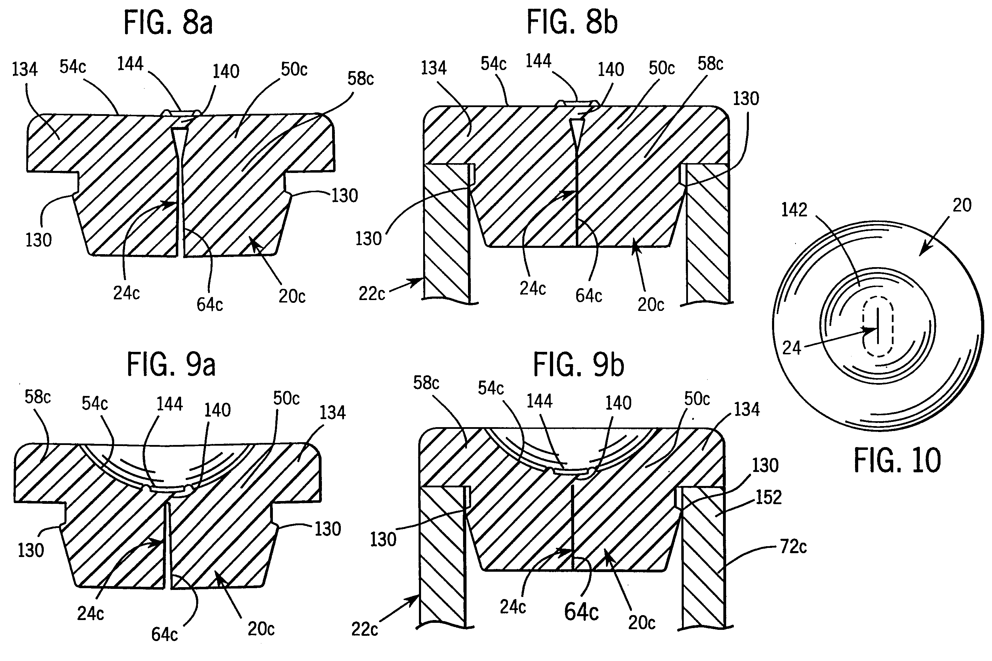

As illustrated in the drawings, a reseal member 20 for use in sealing a fluid access port 22 of a solution container constitutes one of the preferred embodiments of the present invention. The reseal member 20 may be penetrated by a relatively unsharpened blunt cannula 26, for example, a blunt metal or plastic cannula, to pass fluids into the container. The reseal member may alternately be configured for use, as illustrated, at a Y-site of an infusion tubing set. For those features shown in association with a side access port 22b of the solution container which are similar to the features of the down port 22, the features are designated with a "b", for example, reseal member 20b. For those embodiments illustrated in association with an access port 22c of the solution container, also referred to herein as a solution vial (Figures 8a-8b and 9a-9b), which are similar to the features of the down port 22, the features are designated with a "c", for example, reseal member 20c.

The reseal member 20 is provided with a region 24 (Figure 10) which exhibits a relatively reduced resistance to penetration by a blunt cannula 26. To pass fluids through the reseal member 20, the blunt cannula 26 is passed through the reseal member 20 and the reseal member 20 forms a fluid-tight seal around the blunt cannula 26. Upon withdrawal of the blunt cannula 26, the reseal member 20 reforms a generally fluid-tight seal and substantially prevents the passage of fluids therethrough.

The blunt cannula 26 that is used with the present invention is becoming increasingly prevalent and preferred in the healthcare industry for enhancing the efficiency with which solution are administered to patients. The cannula 26 is comprised of a long, thin shaft 28, for example, a long thin steel shaft, having an axial passage (not shown) therethrough. The end 30 of the shaft 28 is surface finished so as to create a blunt end. The outside diameter of the shaft 28 is small, approximately 1,3-1,8 mm (.050-.070 of an inch). The smooth, blunt end 30 of the cannula 26 is highly effective in preventing a user from inadvertently being stuck with the end 30 of the cannula 26. Furthermore, the smooth end 30 prevents the blunt cannula 26 from tearing the interior of the reseal member 20 and desirably acts to prevent the cannula 26 from creating particulate when the blunt cannula 26 is passed through the reseal member 20.

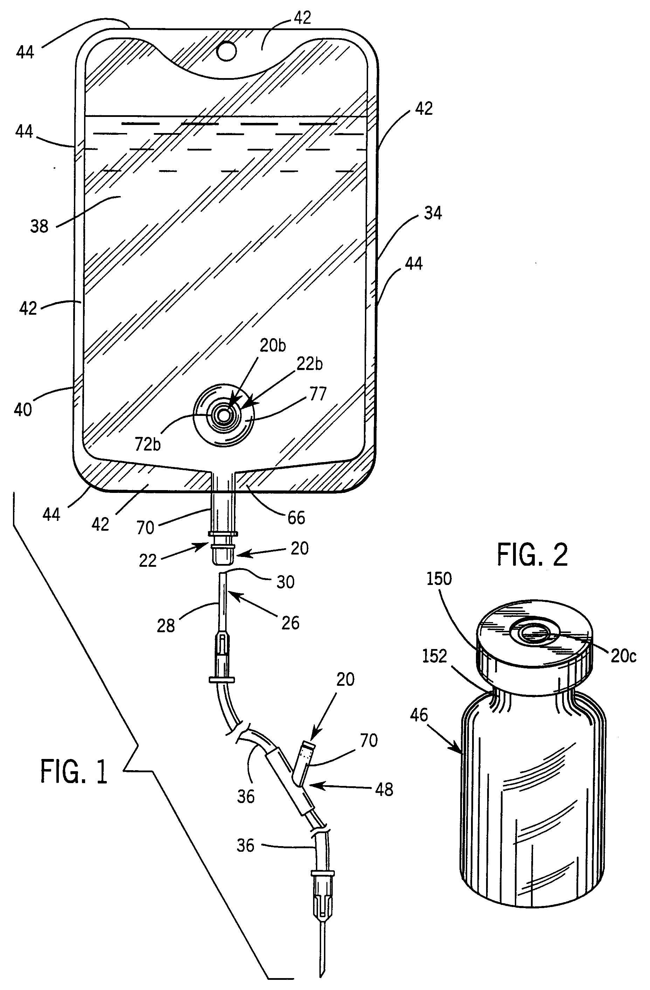

The novel reseal member 20 of the present invention is used to create a fluid tight seal in a fluid access port 22, such as a port 22 in a thin, flexible container 34, typically comprising a vinyl intravenous solution container as illustrated in Figure 1, also referred to herein as an "I.V. bag." It is preferable to allow fluids to be passed through the reseal member 20, so fluids may be removed from the container 34, or be added to and mixed with the fluids in the container 34. Alternatively, the reseal member 20 can be used in a solution vial 46, as shown in Figure 2, or in a Y-site 48 of an infusion tubing set, as shown in Figure 1.

Vinyl I.V. bag 34 and its attachable plastic tubing 36 are of well known constructions and as such, will not be described in detail herein. Briefly, as shown in Figure 1, the I.V. bag 34 includes two plastic sheets 38, 40 bonded together by a heat seal 42 along the edges 44 of the sheets 38,40. Tubing 36, having an axial passage (not shown) therethrough, is attached to the bag 34 by a blunt cannula 26 that is inserted through a reseal member 20. The Y-site 48 may be included along the length of the tubing 36.

In each of the embodiments of Figures 3-7, 8a-8b, and 9a-9c, the reseal member 20 (designated 20c in the embodiments of Figures 8a-8b and 9a-9b) has a forward end portion 50 and rearward end portion 52, each having a face 54, 56 respectively, and takes the form of a generally cylindrically shaped body 58 which defines a length and a radius. The body 58 includes a region 24 which exhibits a relatively reduced resistance to penetration by a blunt cannula 26 relative to the remaining area of the forward end portion 50. This region 24 can include a preformed partial slit 60, a preformed full slit 62 which separates the body 58 into two pieces, a molded hole or recess 64, or a combination of these means, as will be described in detail herein. The molded hole or recess 64 may take one of many forms as illustrated herein without departing from the scope of the invention. When the novel reseal member 20 of the present invention is used in combination with a blunt, metal cannula 26, a user only needs to exert a minimal amount of force, for example, approximately three pounds of force, to insert the blunt cannula 26 through the reseal member 20.

The reseal member 20 is made of a soft elastomeric material, for example, a soft gum rubber or a soft synthetic elastomeric material. Since the reseal member 20 is made of this material, the body 58 of the reseal member 20 is easily displaced by the shaft 28 of the blunt cannula 26 as the cannula 26 passes through the reseal member 20.

In reference to Figure 1, the fluid access port 22 may be housed in a bottom portion 66 of the container 34, in the side of the container 34 or in a Y-site 48 of an infusion tubing set. The reseal member is typically positioned in association with a cylindrical passage 70 of the solution container or Y-site.

The fluid access port 22 is best illustrated in Figures 3-7. It will be understood that features of these embodiments of the present reseal member can be incorporated into a construction suitable for use with a Y-site, a side access port, or a solution vial. The fluid access port 22 is preferably made of a flexible, plastic material and includes a generally cylindrical peripheral wall 72 with a cylindrical, axial passage 74 therethrough. The port 22 includes an annular shoulder 76 around the circumference of the wall 72 at a predetermined distance from an end of the wall 72. The port 22 also includes an integral thin, flexible plastic membrane 78 on the interior circumference of the passage 74 to provide an additional barrier means. The port 22 may also include circumferentially spaced lands 80 (only one is shown for clarity) for molding purposes.

To insert the port 22 into the passage 70, an end portion of the wall 74 is inserted into the passage 70 until the annular shoulder 76 generally abuts the end of the passage 70. Thus, the annular shoulder 76 prevents the fluid access port 22 from being completely inserted into the passage 70. The interior diameter of the passage 70 and the exterior diameter of the fluid access port 22 are approximately the same size so as to create a fluid-tight fit when the fluid access port 22 is inserted into the passage 70. The fluid access port 22 is attached to the passage 70 by appropriate means, such as by solvent bonding to create a mechanical-like bond.

To insert the reseal member 20 into the fluid access port 22, the reseal member 20 is radially compressed and driven into the fluid access port 22 a desired distance by a suitable means. Preferably, the fluid access port 22 is made of a flexible, plastic material. Thus, the port 22 flexes as the reseal member 20 is placed therein. The interior diameter of the port 22 and the exterior diameter of the reseal member 20 are approximately the same size so as to create a fluid-tight fit when the reseal member 20 is inserted into the port 22.

Alternatively, as stated above, the reseal member 20b may be housed in a side access port 22b. Side access ports are well known in the art and will only be described briefly herein. If a side access port 22b is used to house the reseal member 20b, the plastic solution container 34 includes a circular aperture (not shown) in the side 38 of the container 34. The side port 20b includes a generally cylindrical peripheral wall 72b with a cylindrical, axial passage (not shown) therethrough. The side port 20b includes an integral annular shoulder 77 around the circumference of the wall 72b at the rear end of the wall 72b. The side port 22b also includes an integral thin, flexible, plastic membrane (not shown) on the interior circumference of the axial passage. To attach the side port 22b to the container 34, the axial passage and the aperture are aligned and the integral annular shoulder 77, is attached to the container 34 by appropriate means, such as by heat sealing or by solvent bonding to create a mechanical-like bond.

When the blunt cannula 26 is inserted through the reseal member 20, the body 58 of the reseal member 20 is displaced around the cannula 26, and a fluid-tight seal is formed around the cannula 26 due to the natural resiliency of the rubber material. Thus, fluids are generally prevented from leaking through the reseal member 20. The diameter of a blunt cannula 26 is small and creates a small passage (not shown) through the reseal member 20 when the blunt cannula 26 is inserted. After the blunt cannula 26 has been completely inserted through the reseal member 20 and membrane 78, fluids can be passed into the container 34 or removed from the container 34, or into tubing 36 if the reseal member 20 is provided in Y-site 48. When the blunt cannula 26 is withdrawn, the reseal member 20 reforms a generally fluid-tight seal due to the natural resiliency of the elastomeric material such as rubber and fluids are substantially prevented from leaking therethrough.

As shown in Figures 4, 6 and 7, the end 82 of the fluid access port 22 may be angled inwardly around its circumference. This angled end 82 helps to insure that the reseal member 20 remains seated and effectively held captive within the port 22. Alternatively, as shown in Figures 3 and 5, the end 84 of the fluid access port 22 may be straight. Also, as shown in Figures 3, 4 and 7 the reseal member 20 may be spaced a predetermined distance from the membrane 78. Alternatively, the reseal member 20 may abut the membrane 78 as shown in Figures 5 and 6. It is to be understood that any of the embodiments may include an angled end or a straight end, or may be spaced or abut the membrane without departing from the scope of this invention.

As shown in Figure 4, the region 24 that exhibits a relatively reduced resistance to penetration by the blunt cannula 26 relative to the remaining area of the end portion 50 includes a preformed axially extending slit 62. The slit 62 extends axially along the entire length and diametrically of the body 58 thus separating the body 58 into two separate pieces 86, 88. To form this slit 62, the body 58 is molded as two symmetrical halves 86, 88, by conventional molding techniques, with each half 86, 88 having a semi-circular cross-section.

The embodiment of Figure 5 is similar to the embodiment of Figure 4 except the fluid access port 22 includes a tubular, vinyl sleeve or jacket 90 on the port 22 interior that is generally cylindrical with an axial passage 92 therethrough. The interior diameter of the axial passage 92 and the exterior diameter of the reseal member 20 are approximately the same so as to create a fluid-tight seal when the reseal member 20 has been inserted into the jacket 90. The vinyl jacket 90 is bonded to the interior of the port 22 by appropriate means, such as solvent bonding. The reseal member 20 is inserted into the vinyl jacket 90 by appropriate means.

In the embodiments shown in Figures 4 and 5, since the region 24 includes a full length slit 62, the blunt cannula 26 is easily inserted through the reseal member 20. To insert the cannula 26, the user places the blunt end 30 of the cannula 26 against the reseal face 54 and pushes the cannula 26 through the reseal member 20. During insertion of the cannula 26, the body 58 compresses slightly thereby locally widening the slit 62 to allow the blunt, metal cannula 26 to pass therethrough. Due to the natural resiliency of the gum rubber, the body 58 forms a generally fluid-tight seal around the shaft 28 of the cannula 26. When the cannula 26 is removed, the body 58 is decompressed and a generally fluid-tight seal is reformed in the reseal member 20.

It should be noted, for example, in Figures 4 and 5, that the portions of the reseal member in respect to the slit can be made to have contrasting colors or shades to facilitate location of the slit visually. A similar consideration is applicable to Figure 6 wherein, for example, ledge 95 can be transparent whereas piece 94 can be opaque.

In Figure 6, the region 24 again includes a preformed full slit 62 and the reseal member 20 is comprised of two separate pieces 94, 96. However, in this embodiment, the two pieces 94, 96 are nonidentical and can be formed by conventional molding techniques. The slit 62 extends axially and diametrically from the rearmost or distal end of the body 58 to a predetermined position near the exposed or proximal end of the body 58 and across∼the diameter of the body 58. At the portion of the slit 62 near the exposed end of the body 58, the slit 62 extends radially. Thus, as can be seen in Figure 6, the piece 96 generally has a cross-sectional shape of an L. Piece 96 includes a thin, solid ledge 95 at the forward end portion 50 of the reseal body 58 that overlaps piece 94.

In the embodiment shown in Figure 7, the reseal member 20 is made of one integral piece. and the region 24 includes a preformed partial slit 60. The partial slit 60 extends axially from the rearmost end of the reseal member 20 to a predetermined position near the exposed end of the body 58 and also across the diameter of the body 58. To form a partial slit 60, the two halves of the reseal body can be integrally formed, with a thin portion 98 joining the halves, as shown in Figure 7. The halves can then be urged together, with the portion 98 acting as a hinge. Thin portion 98 of the reseal member 20 that is forward of the slit 60 remains as one continuous piece.

In Figure 3, the body 58 of the reseal member 20 further includes a thin, integral annular wall 100 that is connected to the forward end portion 50 of the body 58 by a thin, integral shoulder 102. A circular passage or recess 104 is created between the wall 100 and the body 58. When the reseal member 20 is attached to the fluid access port 22, an end portion 106 of the port 22 is located between the annular wall 100 and the body 58 with the shoulder 102 extending over the end 84 of the port 22. In this embodiment, the shoulder 102 extends over the end 84 of the port 22.

In Figure 3, the region 24 includes a molded hole or recess 64 and a preformed partial slit 60. The recess 64 extends axially from the rearmost end of the body 58 and is formed by conventional molding techniques. The partial slit 60 extends forwardly of the recess 64 in the axial direction. To form the partial slit 60, the gum rubber may be prestressed on anvil and then lanced. The partial slit 60 extends partially across the diameter of the body 58 and terminates at a predetermined distance from the forward end of the body 58 thereby defining a thin, continuous piece 108 at the forward end portion 50 similar to that of the embodiment shown in Figure 7.

To insert the blunt cannula 26, in the embodiments of Figures 3, 6 and 7 as shown, the user places the cannula 26 against the face 54 of the reseal member 20 and pushes the cannula 26 through the reseal member 20. The solid ledge 95 or continuous portion 98, 108 of the reseal member 20 must be penetrated by the blunt cannula 26, but since the reseal member 20 is made of a soft rubber material, the ledge 95 or portion 98, 108 is easily penetrated and displaced around the cannula 26. Furthermore, since the ledge 95 or continuous portion 98, 108 is thin, a user need only apply minimal force to penetrate the body 58.

Once the cannula 26 passes through the ledge 95 or continuous portion 98, 108, the cannula 26 travels along the partial slit 62 and then through the membrane 78. In the embodiment shown in Figure 3, the cannula 26 will also travel through the recess 64 before passing through the membrane 78. As explained hereinabove, as the cannula 26 travels through the body 58, the body 58 is compressed and then forms a generally fluid-tight seal around the cannula 26.

The reseal member 20, designated 20c in the embodiments of Figures 8a-8b and 9a-9b, may also be used in a solution vial 46 (shown in Figure 2). The access port 22c is defined by the neck portion 152 of the container which provides a generally cylindrical peripheral wall 72c within which the reseal member 20c is positioned. An opening in a ferrule cap 150, as shown in Figure 2, of the container 46 provides access to the reseal member 20c. The reseal member 20c is placed beneath the ferrule cap 150 (not shown in Figures 8b and 9b for clarity). Figures 8a and 9a illustrate the reseal member 20c prior to insertion into the associated port, while Figures 8b and 9b illustrate the reseal member 20c after compression and insertion into the access port.

The embodiment of the reseal member 20c illustrated in Figure 8a-8b includes a generally cylindrical body 58c similar to that of Figures 3-7. The body 58c also includes an annular ridge 130 along the length of the body 58c. This annular ridge 130 creates a radial fluid-tight seal with the interior of the port 22c when the body 58c of the reseal member 20c is deformed and inserted into the fluid access port 22c. The body 58c includes an annular shoulder 134 extending from the forward end portion 50c of the body 58c that abuts the end of the port 22c. The region 24c includes an axially extending shaped molded hole or recess 64c which can be formed by conventional molding techniques. The shaped molded recess 64c does not extend the full length of the body 58c. Thus, a solid, continuous portion 140 is forward of the recess 64c. Reseal member 20c can be formed generally as described above in connection with the reseal member shown in Figure 7, wherein the two halves of the reseal member are molded integrally with each other (Figure 8a), then urged together to define the slit-like recess 64c (Figure 8b).

The embodiment illustrated in Figures 9a-9b is similar to the reseal member 20c shown in Figures 8a-8b except that the front end portion 50c of the body 58c includes a dished (*i.e*., concave) face 54c. The dished face 54c facilitates the swabbing of the reseal member 20c with an anti-microbacterial agent or the like before inserting the blunt, metal cannula 26 therethrough. The region 24c also includes an axially extending shaped molded hole or recess 64c. The shaped molded recess 64c does not extend the full length of the body 58c. Thus, a thin, solid, continuous portion 140 is forward of the recess 64c. As in the embodiment of Figures 8a-8b, the reseal member 20 is formed in a configuration as shown in Figure 9a, and is then deformed for insertion into and frictional engagement with the access port 22c, as shown in Figure 9b. A slit-like recess 64c is thus provided.

To insert the blunt cannula 26 into the embodiments shown in Figures 8a-8b and 9a-9b, the user places the cannula 26 against the face 54c of the reseal member and pushes the cannula 26 through the reseal member 20c. The solid portion 140 of the reseal member must be penetrated by the cannula 26, but since the reseal member 20c is made of a soft rubber material, the solid portion 140 is easily penetrated and displaced around the cannula 26. Furthermore, since the solid portion 140 is thin, a user need only apply minimal forces. Once the cannula 26 is inserted through the reseal member 20c, the body 58c of the reseal member 20c forms a generally fluid-tight seal around the blunt cannula 26.

It is contemplated the embodiments shown in Figures 4 and 5 only be used in applications such as at a Y-site 48 of an infusion tubing set which is "sold dry." That is, the reseal member 20 is preferably not wetted until the tubing set is actually used. The embodiments shown in Figures 3, 6,7, 8a-8b and 9a-9b may also be used in a Y-site. However, these embodiments are also suitable for "wet application" in a solution container 34, 46.

As shown in Figure 10, the region 24 creates an area 142 that, if penetrated by a blunt cannula 26, will allow the blunt cannula 26 to be inserted into the reseal member 20. This is also commonly referred to as a "sweet spot." Due to the fact that the preformed slit 60, 62 or recess 64 creates this area 142, a user can insert the blunt cannula 26 into any point within the area 142. If the blunt cannula 26 is inserted into this area 142, the blunt cannula 26 will pass through the preformed slit 60, 62 or recess 64 (or both) to form the passage.

One feature of note is that the reseal member 20 may include a raised ridge-like projection to provide a target 144 on the front end portion 50 of the body 58, as illustrated in Figures 3, 8a-8b and 9a-9b. The target 144 aids a user in inserting a blunt cannula 26 into an area that will cause the blunt cannula 26 to be passed through the preformed slit 60, 62 or recess 64 (or both) of the reseal member 20.

Another feature of note is that in the embodiments that include two molded pieces, as illustrated in Figures 4, 5 and 6, each molded piece may be made of a contrasting or different color. This aids a user in properly inserting the blunt, metal cannula 26 into the reseal member 20.

While several embodiments have been disclosed above, it is to be understood that any of the above embodiments can be easily modified for use in a side port, a down port, of a solution having a ferrule cap container or in a Y-site of an infusion tubing set. Furthermore, it is envisioned that more than one preformed slit may be used in the reseal.

From the foregoing, it will be observed that numerous modifications and variations can be effected without departing frog the scope of the present invention as defined in the claims. It is to be understood that no limitation with respect to the specific embodiments is intended or should be inferred. The disclosure is intended to cover by the appended claims all such modifications as fail within the scope of the claims.

Where technical features mentioned in any claim are followed by references signs, those reference signs have been included for the sole purpose of increasing the intelligibility of the claims and accordingly, such reference signs do not nave any limiting effect on the scope of each element identified by way of example by such reference signs.

## Claims

1. A fluid access port assembly readily penetrable by a blunt cannula (26) comprising:
a fluid port (22), said fluid port (22) being defined by a generally cylindrical peripheral wall (72) having open ends, and
a reseal member (20) comprising an elastomeric body (58) positionable within said peripheral wall (72) defining said fluid port (22) in fluid tight relationship with said peripheral wall (72), said elastomeric body (58) comprising:
- a forward end portion (50) with a front face (54) positioned generally at one of the open ends of said peripheral wall (72) to permit insertion of an associated relatively unsharpened blunt cannula (26) through said reseal member (20),
- a rearward end portion (52) with a rear face (56) positioned within said peripheral wall (72),
- an axially extending slit (60), and
- an axially extending recess (64) extending from said rear face (56) into said rearward end portion (52), said slit (60) extending from said recess (64) into said forward end portion (50), whereby a region (24) of said elastomeric body (58) including said axially extending recess (64) and said slit (60) exhibits a relatively reduced resistance to penetration by said blunt cannula (26) relative to the remaining area of said elastomeric body (58),
CHARACTERIZED IN THAT said slit (60) terminates at a predetermined distance from said front face (54).

2. The fluid access port assembly as defined in claim 1 further comprising a solution container (34), wherein said fluid port (22) is attached to said solution container (34).

3. The fluid access port assembly as defined in claims 1 or 2, wherein said peripheral wall (72) includes a thin, flexible, puncturable membrane (78) housed within said peripheral wall (72), said membrane (78) located between said elastomeric body (58) and one of said open ends of said fluid port (22).

4. The fluid access port assembly as defined in one or more of claims 1-3, wherein said region (24) of said elastomeric body (58) includes a target (144) at said forward end portion (50).

5. The fluid access port assembly as defined in one or more of claims 1-4, wherein said elastomeric body (58) includes an annular shoulder (102) extending outwardly from said forward end portion (50) and an annular wall (100), said annular wall (100) extending substantially concentrically with said elastomeric body (58) so as to define a circumferential recess (104) between said annular wall (100) and said elastomeric body (58), said circumferential recess (104) engagingly receiving said peripheral wall (72).

6. The fluid access port assembly as defined in one or more of claims 1-5, wherein said elastomeric body (58) comprises two integrally joined halves positioned adjacent each other, and joined to each other by a relatively thin portion of said elastomeric body (58) through which said blunt cannula (26) is insertable.

7. The fluid access port assembly as defined in one or more of claims 1-6, wherein said forward end portion (50) is dished to facilitate swabbing said forward end portion (50) with an antimicrobacterial agent before the insertion of said blunt cannula (26).

## Patentansprüche

1. Fluidzugangsöffnungs-Anordnung, die von einer stumpfen Kanüle (26) einfach durchdrungen werden kann, und die folgendes umfaßt:
eine Fluidöffnung (22), wobei die Fluidöffnung (22) durch eine allgemein zylindrische Außenwand (72) definiert ist, die offene Enden aufweist, und
ein wiederabdichtendes Glied (20), das einen Elastomerkörper (58) umfaßt, der innerhalb der Außenwand (72), die die Fluidöffnung (22) definiert, auf fluiddichte Weise mit der Außenwand (72) angebracht werden kann, wobei der Elastomerkörper (58) folgendes umfaßt:
- einen vorwärtigen Endabschnitt (50) mit einer vorderen Fläche (54), die allgemein an einem der offenen Enden der Außenwand (72) angebracht ist, um die Einführung einer dazugehörigen vergleichsweise ungespitzten, stumpfen Kanüle (26) hindurch das wiederabdichtende Glied (20) zu erlauben,
- einen rückwärtigen Endabschnitt (52) mit einer hinteren Fläche (56), die innnerhalb der Außenwand (72) angebracht ist,
- einen sich achsial erstreckenden Schlitz (60), und
- einen sich axial erstreckender Einschnitt (64), der sich von der hinteren Fläche (56) in den rückwärtigen Endabschnitt (52) hinein erstreckt, wobei sich der Schlitz (60) von dem Einschnitt (64) aus in den vorwärtigen Endabschnitt (50) erstreckt, wodurch ein Bereich (24) des Elastomerkörpers (58), der den sich achsial erstreckenden Einschnitt (64) und den Schlitz (60) einschließt, im Verhältnis zum verbleibenden Bereich des Elastomerkörpers (58) einen ziemlich verminderten Widerstand gegenüber dem Eindringen der stumpfen Kanüle (26) aufweist,
DADURCH GEKENNZEICHNET, DAß der Schlitz (60) in einem vorbestimmten Abstand von der vorderen Fläche (54) endet.

2. Fluidzugangsöffnungs-Anordnung nach Anspruch 1, die weiterhin einen Lösungsbehälter (34) umfaßt, worin die Fluidöffnung (22) am Lösungsbehälter (34) angebracht ist.

3. Fluidzugangsöffnungs-Anordnung nach Anspruch 1 oder 2, worin die Außenwand (72) eine dünne, flexible durchstechbare Membran (78) umfaßt, die innerhalb der Außenwand (72) untergebracht ist, wobei sich die Membran (78) zwischen dem Elastomerkörper (58) und einem der offenen Enden der Fluidöffnung (22) befindet.

4. Fluidzugangsöffnungs-Anordnung nach einem oder mehreren der Ansprüche 1-3, worin der Bereich (24) des Elastomerkörpers (58) am vorwärtigen Endabschnitt (50) ein Target (144) umfaßt.

5. Fluidzugangsöffnungs-Anordnung nach einem oder mehreren der Ansprüche 1-4, worin der Elastomerkörper (58) eine ringförmige Schulter (102) umfaßt, die sich außerhalb des vorwärtigen Endabschnitts (50) und einer ringförmigen Wand (100) erstreckt, wobei sich die ringförmige Wand (100) im wesentlichen konzentrisch mit dem Elastomerkörper (58) erstreckt, um zwischen der ringförmigen Wand (100) und dem Elastomerkörper (58) einen umfänglich verlaufenden Einschnitt (104) zu definieren, wobei der umfänglich verlaufende Einschnitt (104) die Außenwand (72) eingreifend aufnimmt.

6. Fluidzugangsöffnungs-Anordnung nach einem oder mehreren der Ansprüche 1-5, worin der Elastomerkörper (58) zwei benachbart zueinander angebrachte, integral verbundene Hälften umfaßt, und die miteinander über einen ziemlich dünnen Abschnitt des Elastomerkörpers (58) verbunden sind, durch den die stumpfe Kanüle (26) eingeführt werden kann.

7. Fluidzugangsöffnungs-Anordnung nach einem oder mehreren der Ansprüche 1-6, worin der vorwärtige Endabschnitt (50) einwärts gekrümmt ist, um das Abwischen des vorwärtigen Endabschnitts (50) mit einem antimikrobakteriellen Mittel zu erleichtern, bevor die stumpfe Kanüle (26) eingeführt wird.

## Revendications

1. Assemblage d'orifice d'accès à un fluide facilement pénétrable par une canule émoussée (26) comprenant :
un orifice de fluide (22), ledit orifice de fluide (22) étant défini par une paroi périphérique en générale cylindrique (72) présentant des extrémités ouvertes, et
un élément de refermeture étanche (20) comprenant un corps élastomère (58) pouvant être positionné à l'intérieur de ladite paroi périphérique (72) définissant ledit orifice de fluide (22) dans une relation étanche au fluide avec ladite paroi périphérique (72), ledit corps élastomère (58) comprenant :
- une partie d'extrémité avant (50) avec une face avant (54) disposée en général à l'une des extrémités ouvertes de ladite paroi périphérique (72) pour permettre l'insertion d'une canule émoussée, relativement non pointue, associée (26) à travers ledit élément de refermeture étanche (20),
- une partie d'extrémité arrière (52) avec une face arrière (56) disposée à l'intérieur de ladite paroi périphérique (72),
- une fente s'étendant axialement (60), et
- un creux s'étendant axialement (64) s'étendant à partir de ladite face arrière (56) dans ladite partie d'extrémité arrière (52), ladite fente (60) s'étendant à partir dudit creux (64) dans ladite partie d'extrémité avant (50), une région (24) dudit corps élastomère (58) comprenant ledit creux s'étendant axialement (64) et ladite fente (60) présente par là une résistance relativement réduite à la pénétration par ladite canule émoussée (26) par rapport à la surface restante dudit corps élastomère (58),
CARACTERISE EN CE QUE ladite fente (60) se termine à une distance prédéterminée de ladite face avant (54).

2. Assemblage d'orifice d'accès à un fluide selon la revendication 1, comprenant en outre un récipient de solution (34), dans lequel ledit orifice de fluide (22) est fixé audit récipient de solution (34).

3. Assemblage d'orifice d'accès à un fluide selon la revendication 1 ou 2, dans lequel ladite paroi périphérique (72) comprend une mince membrane flexible, perforable (78) logée à l'intérieur de ladite paroi périphérique (72), ladite membrane (78) est disposée entre ledit corps élastomère (58) et une desdites extrémités ouvertes dudit orifice de fluide (22).

4. Assemblage d'orifice d'accès à un fluide selon l'une ou plusieurs quelconques des revendications 1-3, dans lequel ladite région (24) dudit corps élastomère (58) comprend une cible (144) sur ladite partie d'extrémité avant (50).

5. Assemblage d'orifice d'accès à un fluide selon l'une ou plusieurs quelconques des revendications 1-4, dans lequel ledit corps élastomère (58) comprend un épaulement annulaire (102) s'étendant vers l'extérieur à partir de ladite partie d'extrémité avant (50) et une paroi annulaire (100), ladite paroi annulaire (100) s'étendant pratiquement concentriquement audit corps élastomère (58) afin de définir un creux circonférentiel (104) entre ladite paroi annulaire (100) et ledit corps élastomère (58), ledit creux circonférentiel (104) recevant ladite paroi périphérique (72) par enclenchement.

6. Assemblage d'orifice d'accès à un fluide selon l'une ou plusieurs quelconques des revendications 1-5, dans lequel ledit corps élastomère (58) comprend deux moitiés jointes en une pièce, disposées l'une à côté de l'autre et jointes l'une à l'autre par une partie relativement mince dudit corps élastomère (58) à travers laquelle ladite canule émoussée (26) peut être insérée.

7. Assemblage d'orifice d'accès à un fluide selon l'une ou plusieurs quelconques des revendications 1-6, dans lequel ladite partie d'extrémité avant (50) est emboutie pour faciliter le nettoyage de ladite partie d'extrémité avant (50) avec un agent antimicrobactérien avant l'insertion de ladite canule émoussée (26).
